# EUROPEAN PATENT APPLICATION

(11) **EP 1 433 847 A1**
(43) Date of publication of application: **30.06.2004**
(21) Application number: 03022984.3
(22) Date of filing: 10.10.2003
(51) Int. Cl.: C12N 15/11

(54) **Method of interfering with gene expression in mammalian post-implantation embryos**

(30) Priority: 10.10.2002 EP 02022922
(71) Applicant: Max-Planck-Gesellschaft zur Förderung der Wissenschaften e.V., Berlin (DE)
(72) Inventor: Huttner, Wieland B., 01309 Dresden (DE); Buchholz, Frank, 01309 Dresden (DE); Calegari, Federico, 01326 Dresden (DE); Haubensak, Wulf Eckhard, 01307 Dresden (DE)
(74) Representative: VOSSIUS & PARTNER

(57) **Abstract**

The present invention relates to a method of interfering with gene expression in a mammalian post-implantation embryo comprising the step of introducing siRNA molecules or shRNA molecules or DNA molecules encoding siRNA or shRNA molecules wherein said siRNA molecules or said shRNA molecules have a region of identity or of substantial identity of at least 15 nucleotides with the gene(s) the expression of which is to be interfered with into cells of said post-implantation embryo. Additionally, the present invention pertains to a method of performing a genomic-wide functional screen comprising introducing variety of siRNA molecules or shRNA molecules or DNA molecules encoding a variety of siRNA or shRNA molecules wherein said different siRNA or shRNA molecules have a region of identity or substantial identity of at least 15 bp with different genes into a mammalian post-implantation embryo.

## Description

The present invention relates to a method of interfering with gene expression in a mammalian post-implantation embryo comprising the step of introducing siRNA molecules or shRNA molecules or DNA molecules encoding siRNA or shRNA molecules wherein said siRNA molecules or said shRNA molecules have a region of identity or of substantial identity of at least 15 nucleotides with the gene(s) the expression of which is to be interfered with into cells of said post-implantation embryo. Additionally, the present invention pertains to a method of performing a genomic-wide functional screen comprising introducing variety of siRNA molecules or shRNA molecules or DNA molecules encoding a variety of siRNA or shRNA molecules wherein said different siRNA or shRNA molecules have a region of identity or substantial identity of at least 15 bp with different genes into a mammalian post-implantation embryo.

In this specification, a number of documents is cited. The disclosure content of these documents including manufacturers' manuals, is herewith incorporated by reference in its entirety.

Understanding the function of genes requires methods that allow manipulation of gene expression. The discovery that double-stranded RNA (dsRNA) can be used for RNA interference (RNAi) in certain invertebrates and plants has allowed the systematic analysis of gene function in these organisms (1-6). In most mammalian cells, however, dsRNA triggers the interferon response, which leads to general shutdown of gene expression and/or cell death (7). dsRNA is therefore not useful for gene function analysis in most mammalian cells. In contrast, short interfering RNA (siRNA) does not trigger the interferon response, whilst being an efficient mediator of posttranscriptional gene silencing in mammalian cell lines (8-11).

To understand gene function in a physiological context, however, methods are required that allow their investigation in complex systems such as the whole animal. In the mouse, gene function can be studied through gene targeting using homologous recombination in embryonic stem cells. However, the generation of gene knock-out mice is cost-, time-, and labor-intensive. Accordingly, there is a need in the art to provide alternative systems that fulfil the same or similar functions in an shorter time frame, with a reduced cost factor and that are less labor-intensive. Said need in the art forms the technical problem underlying the present invention, the solution of which is achieved by providing the embodiments characterized in the claims.

Thus, the present invention relates to a method of interfering with gene expression in a mammalian post-implantation embryo comprising the step of introducing siRNA molecules or shRNA molecules or DNA molecules encoding siRNA or shRNA molecules wherein said siRNA molecules or said shRNA molecules have a region of identity or of substantial identity of at least 15 nucleotides with the gene(s) the expression of which is to be interfered with into cells of said post-implantation embryo.

The term "interfering with gene expression" in accordance with the present invention refers to a block or reduction of gene expression at the posttranscriptional level in particular by the destabilization of the mRNA and translational inhibition. This effect seems to be mediated by the RISC complex (RNA-induced silencing complex). RISC contains a nuclease (DICER) that cleaves dsRNA into ca. 21 bp pieces that are deemed the effective molecules in RNAi. The interference has the effect that at least translation is reduced by at least 50%, preferably at least 75%, more preferred at least 90%, still more preferred at least 95%, such as at least 98% and most preferred at least 99%.

The term "mammalian post-implantation embryo" relates to the stage from which long dsRNA (<40 bp) trigger the interferon response in fertilized eggs until birth. Alternatively, this term means in accordance with the invention the stage from implantation of the embryo into the wall of the uterus until birth. In humans, this stage starts approximatively after 10 days p.c. In mice, it starts after about 4.5 days p.c.

The term "siRNA" (small interfering RNA) refers to ribonucleic acid molecules that have a sequence of identity or substantial identity with a target gene the expression of which is to be interfered with. The siRNA molecules are essentially double-stranded but may comprise 3' or 5' overhangs. They may also comprise sequences that are not identical or essentially identical with the target gene but these sequences must be located outside of the sequence of identity. The sequence of identity or substantial identity is at least 14 and more preferably at least 19 such as 21 nucleotides long. It preferably does not exceed 23 nucleotides. Optionally, the siRNA comprises two regions of identity or substantial identity that are interspersed by a region of non-identity.

"Identity" is defined by 100% match of the sense strand of the siRNA to the targeted mRNA. "Substantial identity" is defined by 1-2 mismatches of the sense strand of the siRNA to the targeted mRNA or 10-15% over the total length of siRNA to the targeted mRNA mismatches within the region of identity. Said mismatches may be the result of a nucleotide substitution, addition, deletion or duplication etc. dsRNA longer than 23 but not bigger than 40 bp may also contain 3-4 mismatches. Nonidentity is a sequence that has more than 15% mismatches with the sense strand of the siRNA to the targeted mRNA.

The term "shRNA" (short hairpin RNA) refers to RNA molecules that fold into a stem-loop structure with 3' UU-overhangs with, e.g. a length of about 21 bp.

In accordance with the present invention and as a more convenient alternative to the generation of transgenic animals, the possibility of using siRNA to trigger RNAi in mammals was explored, focussing on post-implantation embryos. As a model system apt to allow substantive conclusions with regard to other mammals such as primates including humans, the mouse was employed. It was surprisingly found that RNAi functions when tested in such post-implantation embryos. This finding has important implications on functional studies of gene expression in whole animals. In comparison to adult mammals, different genes and different gene pathways are used during embryonic development. In addition, many important functions for life, including defense mechanism against pathogens, are provides by the mother. Therefore, it was not to be expected without further ado that an RNAi response would be functioning in post-implantation embryos. The present invention shows, that almost every cell within an embryonic tissue or a region of a tissue can be targeted and is amenable for gene silencing. With this method, genes and gene pathways important for mammalian development can be rapidly identified. Furthermore, this technique opens a novel approach for treatment of genetic diseases that manifest during embryonic development.

Whole-embryo culture supports the normal development of mouse postimplantation embryos for up to two days in vitro (12). In addition, whole-embryo culture can conveniently be combined with various methods of introducing foreign DNA into cells of the developing embryo, including electroporation; see Oback, B., Cid-Arregui, A. & Huttner, W.B. (2000) in Viral Vectors: Basic Science and Gene Therapy, ed. Garcia-Carranca, (Eaton Publishing, Natick. MA) pp. 277-293; Osumi N, Inoue T. (2001) Methods. 2001 May; 24(1):35-42; Takahashi M, Sato K, Nomura T, Osumi N. (2002) Differentiation. 2002 Jun; 70(4-5):155-62. Using the neuroepithelium of E10 mouse embryos as a model target tissue, the use of electroporation as a possible means of introducing siRNA was investigated, specifically endoribonuclease-prepared siRNA (esiRNA), into neuroepithelial cells in defined regions of the developing central nervous system, in order to achieve tissue- and region-specific RNAi during subsequent development in whole-embryo culture. The experimental results as detailed in the examples allow the conclusion that siRNA (as well as shRNA) can effienciently be used for the specific interference of gene expression. In this way, the expression of desired genes, alone or in combination, may be blocked and their influence in, for example, various developmental or differentiation settings be explored. The introduction of DNA encoding either siRNA or shRNA molecules offers even more versatile opportunities. This is because the RNA molecules may be placed under the control of an inducible promoter. In this way, the specific time point of interference with gene expression may be triggered in accordance with the specific questions posed by the investigator. Alternatively, constitutive promoters may be used.

The present invention can be used for therapeutic interference with target genes in animals or humans. Such interference would be effected in compliance with all relevant laws, guidelines and ethical considerations. For example, RNAi based on the method of the present invention may be applied for the targeting of genes known to be causative of genetic diseases. For instance, embryonic tumors may be treated by siRNA directed against a relevant oncogene. In this way the tumor could be specifically targeted without effecting the normal development of the embryo. The method will also be useful to identify genes important for proper mammalian development. This information could be the starting point for the treatment and prevention of malformations caused by genetic defects.

The method may also increase the productivity of animal farming. For instance, genes may be identified that when regulated lead to healthier or bigger newborns.

In a preferred embodiment of the method of the invention, said mammalian post-implantation embryo is a human embryo, a non-human primate embryo, a dog, cat, pig, cow, horse, donkey, goat, sheep, rabbit, rat or a mouse embryo.

In another preferred embodiment of the method of the invention, said post-implantation embryo is in the stage between implantation in the wall of the uterus and birth. Implantation is the term applied to the process by which the conceptus attaches to the wall of the uterus. In the mouse, implantation into the wall of the uterus happens around day E4.5 p.c. and in humans around day E10 p.c.

A variety of techniques are available in the art that may be used to incorporate the RNA or DNA molecules into the target cells. These include double glycerol-shock transfection protocol, calcium phosphate method, DEAE-dextran transfection, lipofection, genegun, nucleic acid/polymer transfection, TAT/Antp-fusions, simple injection, high pression delivery methods (see, e.g., Lewis et al., Nature Genetics 32(2002), 107-108) and hydrodynamic transfection methods (see, e.g. McCaffrey et al., Nature 418 (2002), 38-39). In accordance with the method of the invention it is preferred that the introduction is effected by injection into the embryo, preferably followed by electroporation or nucleic acid transfection or by any further suitable method as indicated above. Thus, in principle, the introduction may be effected by injection into the embryo without subsequent treatment or injection followed by a treatment to improve uptake or transfer of the siRNA or shRNA into organs, tissues and cells of said post-implantation embryo, including but not limited to treatment by double glycerol-shock transfection protocol, calcium phosphate method, DEAE-dextran transfection, lipofection, Genegun, nucleic acid/polymer transfections, and TAT/Antp-fusions and other viral, lipid or lipid vesicle, non-viral, or polycationic transfection methods. Injection without subsequent treatment may be effected directly into the bloodstream. Electroporation is a technique well established in the art for transporting nucleic acid molecules into cells. Conditions can be adjusted by the skilled artisan to not damage the embryonic cells. Such conditions include electroporation with 5 square electrical pulses of 30V, 50 ms each at 1 s intervals using platinum electrodes (7 mm diameter, 1 cm distance) mounted on plastic tweezers using a BTX ECM830 electroporator. (Other conditions may also work).

siRNA may be prepared by a variety of methods well known in the art including chemical synthesis and in vitro transcription. For the chemical synthesis, e.g. two complementary single stranded RNA molecules are synthesized on a solid phase sytheziser and annealed to form a double stranded siRNA molecule with 2 base pair 3' overhangs. For in vitro transcription the ssRNA molecules are e.g. generated using in vitro transcription kits like the Silencer™ siRNA Construction Kit from Ambion, Austin, Tx. Alternatively, the siRNAs can be generated through in vitro transcription of a short hairpin using the same Silencer™ siRNA Construction Kit. Particularly preferred is a method wherein the siRNA has been prepared by endoribonuclease-digestion. Preferred endoribonucleases are RNAse III type enzymes, including RNAse III from E. coli or other bacteria and RNAse III/DICER enzymes from eukaryotes. This embodiment of the invention has the specific advantage that that screening for effective mediators of mRNA interference, also referred to as silencing throughout this specification is not necessary since the cocktail of different siRNAs usually contains effective molecules. In addition, the use of a mixture of different siRNAs targeted against the same mRNA seems to increase the silencing efficiency (11). This result was corroborated in accordance with the present invention.

It is furthermore preferred in accordance with the method of the present invention that the siRNAs have a maximum length of about 40 bp. This maximum length avoids, on the one hand, the interferon response referred to herein above and on the other hand, is the average upper limit of an endoribonuclease digestion that leads to useful medium length of siRNA molecules. Using 0.2 µg E. coli RNAse III and 100µg dsRNA in a digestion at 20°C typically leads to fragments that range in size from 15 bp to 40 bp with most of the molecules having a size around 21 bp.

An additional preferred embodiment of the present invention relates to a method wherein a variety of siRNA molecules or shRNA molecules or DNA molecules encoding a variety of siRNA or shRNA molecules are introduced wherein different siRNA molecules have a region of identity or of substantial identity of at least 15 bp with different genes. This embodiment of the present invention is particularly suitable to investigate the interplay of different gene products which may or may not be members of a protein cascade or pathway. This method may identify all genes involved in these cascades or pathways and may therefore explain certain gene networks. Possible cascades or pathways include genes involved in oxidative phosphorylation, cell migration, cell size, cell cycle, apoptosis, poliferation, differentiation, endocytosis, angiogenesis, and many more.

In principle, any gene function may be investigated with the method of the present invention. Particularly preferred in accordance with the invention is that the gene(s) the expression of which is interfered with is/are selected from the group of developmental genes or genes involved in the generation of cancer or other diseases. The investigation of the function of developmental genes is suggested in accordance with the present invention in particular when employing non-human embryos. The invention allows the advantageous insertion of various siRNA targeting different genes in the embryonic stage without interfering with the viability of the embryo. In this way, the interplay of the various targeted gene products can be studied. As was pointed out herein above, the analyses can be further refined controlling the expression of the siRNAs or shRNAs from inducible promoters.

Similarly, the exact function of cancer related genes or gene products with other genes/gene products would be studied with non-human embryos whereas the targeted gene silencing for therapeutic purposes would include human embryos, again strictly in line with governmental laws, regulations and ethical considerations etc.

It is also preferred that said mammalian post-implantation embryo is cultured on a mammalian and preferably non-human whole-embryo culture or in the uterus. Whole-embryo culture has proven advantageous in the art since it allows the in vitro cultivation of embryos at least for two days (12). In addition, using this type of culture, the embryo can conviently be manipulated with nucleic acid injection, electroporation and transfection techniques and other techniques of nucleic acid incorporation.

An additional preferred embodiment of the present invention relates to a method wherein said siRNA or said shRNA is chemically modified or derivatised. Appropriate modifications or derivatisations include those whereby the two strands of the double stranded siRNA have been linked together or those in which the ends of the RNA strands or individual bases have been modified by chemical addition of groups including but not limited to a halide atom bound to an alkyl, alkenyl, alkinyl or aryl residue, an alcohol group (primary, secondary, tertiary), an ether group, a carbonyl function (aldehyde or ketone), a carboxylic acid group, a carboxylic anhydride group, a carbamoyl group, a haloformyl group, a cyano group, an ester group including a lactone group, a benzyl, phenyl, tolyl, tosyl, sulfonyl group, an amino group (primary, secondary, tertiary), an isocyanate, a cyanate, a thioisocyanate, a thiocyanate, a carbamate, an azide, a diazo group, and a quinone group; or is an organometallic compound, a sterol moiety(ies)-containing molecule, a β-hydroxy carboxylic acid, an inorganic acid or complex such as a metallocene, a cytokine, a hormone, or an oligopeptide comprising up to 20, preferably 8, 10 or 12, amino acid residues.

The present invention also relates to a method of performing a genomic-wide functional screen comprising introducing a variety of siRNA molecules or shRNA molecules or DNA molecules encoding a variety of siRNA or shRNA molecules wherein said different siRNA or shRNA molecules have a region of identity or substantial identity of at least 15 bp with different genes into a mammalian post-implantation embryo. The generation of a library of esiRNA could be generated from cDNA libraries like the once collected through the IMAGE Consortium (www.rzpd.de); see also Pietu G et al., Genome Res. 1999 Feb; 9(2):195-209. Each clone would be PCR amplified using universal primers carrying a T7 promoter and dsRNA generated using in vitro transcription followed by annealing. The dsRNA would then be digested with RNAse III to form siRNA. Each clone (or pools of clones) could then be tested by injection and electroporation in preferably E10 mouse embryos. Embryos would then be cultured and analysed 1-2 days later for possible developmental defects. Upon the establishment of a mouse tumor model with embryonic cancer, these embryos could be used to identify genes that inhibit tumor growth.

Preferred embodiments of this method of the invention include those that have been detailed in accordance with the method of the invention that has been characterized herein above. These preferred embodiments apply mutatis mutandis to this embodiment of the invention.

The figures show:

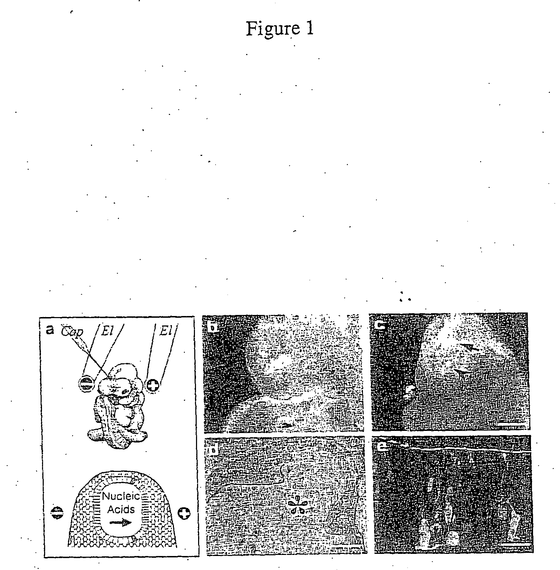

### Figure 1: Approach used for tissue-specific RNAi in postimplantation mouse embryos.

**a** Cartoon illustrating the region-specific injection (Cap, capillary) of siRNA into the lumen of the neural tube of an E10 mouse embryo, with the electroporation electrodes (EI) in the lateral, cathode-right/anode-left orientation (top), and the uptake of siRNA into the left side of the neuroepithelium upon electroporation (bottom). **b-e** Directed uptake of nucleic acids into neuroepithelial cells upon region-specific injection into the lumen of the anterior neural tube of E10 mouse embryos followed by electroporation, exemplified by the use of pEGFP-N2 and expression of GFP upon subsequent whole-embryo culture for 24 h. **b, c** Anterior region of whole unfixed embryos showing GFP expression in the left ventral telencephalon upon injection into the telencephalic neural tube and electroporation using the lateral, cathode-right/anode-left orientation (**b**), and in the dorsal telencephalon (**c**, arrowhead) and dorsal mesencephalon (**c**, arrow) upon injection into the mesencephalic neural tube and electroporation using a cathode-caudal/anode-rostral orientation. Bar in (**c**), 500 µm. **d** Coronal-horizontal cryosection through the mesencephalon of the embryo in (**c**) showing GFP expression in the neuroepithelium (combined phase contrast and fluorescence microscopy). The lumen of the neural tube is indicated by the asterisk. Bar, 200 µm. **e** Higher magnification fluorescence micrograph of a horizontal cryosection through the hindbrain of another embryo upon injection into the rhombencephalic neural tube and electroporation using a cathode-dorsal/anode-ventral orientation, showing GFP expression in individual neuroepithelial cells and neurons derived therefrom. The lumenal surface of the neural tube is indicated by the dashed line. Bar, 10 µm.

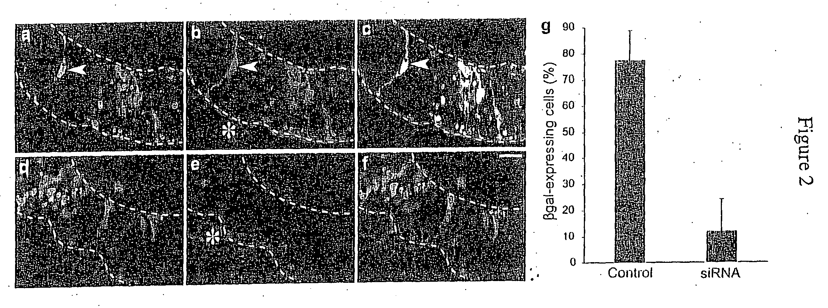

### Figure 2: Specificity of esiRNA-mediated RNAi in the neuroepithelium of postimplantation mouse embryos developing in whole-embryo culture.

E10 mouse embryos were injected, into the lumen of the telencephalic neural tube, with the GFP-expressing plasmid pEGFP-N2 plus the β-gal-expressing plasmid pSVpaXΔ either without (**a-c, g** left column) or with (**d-f, g** right column) β-gal-directed esiRNAs, followed by directed electroporation (lateral, cathode-right/anode-left orientation) and whole-embryo culture for 24 h. **a-f** Horizontal cryosections through the left telencenphalon were analyzed by double fluorescence for expression of GFP (green; **a, d**) and β-gal immunoreactivity (red; **b, e**). Neuroepithelial cells expressing both GFP and β-gal (arrowheads) appear yellow in the merge (**c, f**). Note the lack of β-gal expression in neuroepithelial cells in the presence of β-gal-directed esiRNAs. Upper and lower dashed lines indicate the lumenal (apical) surface and basal border of the neuroepithelium, respectively. Asterisks in (**b**, **e**) indicate the basal lamina and underlying mesenchymal cells, which cross-react with the secondary antibody used to detect β-gal immunoreactivity. Scale bar in (**f**), 20 µm. **g** Quantitation of the percentage of GFP-expressing neuroepithelial cells that also express β-gal without (Control) or with (siRNA) application of □gal-directed esiRNAs. Data are the mean of three embryos analyzed as in (**a-f**); bars indicate S.D.

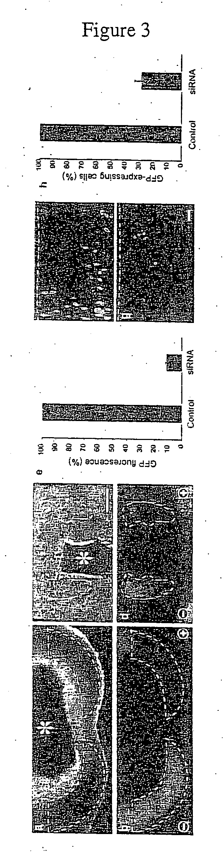

### Figure 3: esiRNA-mediated RNAi of a gene endogenously expressed during the development of postimplantation mouse embryos.

Heterozygous E10 *tis21*^{+/GFP} mouse embryos were injected with GFP-directed esiRNAs into the lumen of the telencephalic (**a, b**) or diencephalic (**c, d, f, g**) neural tube, followed by directed electroporation (lateral, cathode-right/anode-left orientation) and whole-embryo culture for 24 h. **a-d** Low-power dark-field (**a, c**) and fluorescence (**b, d**) micrographs of horizontal vibratome sections through the telencenphalon (**a, b**) and diencephalon (**c, d**). The left half of the brain is on the right side of the panels. Note the silencing of GFP expression in defined regions of the neuroepithelium (dashed lines) in the left, anode-facing half of the embryo. Asterisks in (**a**) and (**c**) indicate the lumen of the neural tube.Scale bar in (**c**), 200 µm. **f, g** Higher magnification fluorescence micrographs of horizontal cryosections through the right (**f,** cathode-facing) and left (**g,** anode-facing) half of the diencephalon at boundary to the telencephalon, prepared from the vibratome section shown in (**d**). Dashed lines indicate the lumenal (apical) surface of the neuroepithelium. Scale bar in (**f**), 20 µm. **e, h** Quantitation of GFP fluorescence (**e**) and GFP-expressing cells (**h**) in the right (cathode-facing) and left (anode-facing) half of the neuroepithelium. Data in (**e**) are the mean of three distinct regions of the neuroepithelium, each from an independent embryo, two of which are the regions shown in (**b**) and (**d**). Data in (**h**) are the mean of two cryosections prepared from the vibratome section shown in (**d**); the region counted was the entire diencephalic neuroepithelium to the boundary to the telencephalon, including the fields shown in (**f, g**). For each embryo/determination, values obtained for the left (anode-facing) half of the neuroepithelium (siRNA) were expressed as percentage of that for the right (cathode-facing) half (Control); the latter was arbitrarily set to 100; bars indicate S.D. (**e**) or the variation of the duplicates from the mean (**h**).

The examples illustrate the invention.

### Example 1: Targeted interference of a model gene in mouse embryos

Methods

### Preparation of esiRNA

esiRNA was prepared as described previously (11). Briefly, ssRNA was obtained from PCR-derived templates carrying T7 and T3 promoters using the MEGAscript kit from Ambion (Austin, TX). The following primers were used: T7-β-gal: 5'-TAATACGACTCACTATAGGGAGAATCGTAATCACCCGAGTGTGA T3- β-gal: 5'-AATTACCCTCACTAAAGGGAGCCCTAATCCGAGCCAGTTTA T7-EGFP: 5'-TTAATACGACTCACTATAGGTGAGCAAGGGCGAGGA T3-EGFP: 5'-TAATTAACCCTCACTAAAGGGTACAGCTCGTCCATGCCGA Annealed dsRNA (100 µg) was digested with 0.2 µg RNAse III for 1 h at 20°C. The sample was mixed with 5 volumes of PN buffer (Qiagen, Hilden, Germany) and loaded onto a QIAquick column (Qiagen, Hilden, Germany). The flow-through, containing dsRNA of 15-40 bp, was precipitated with 0.7 volumes of 2-propanol. The pellet was washed with 750 µl 70% ethanol and dissolved in 1 µM EDTA, 10 µM Tris-HCl pH 8.0, to an RNA concentration of 0.5 µg/µl.

### Whole-embryo electroporation and culture

Manipulation of mouse embryos was performed at room temperature in Dulbecco's PBS containing 10% of heat-inactivated FCS and 100 U/ml of penicillin/streptomycin. E10 embryos were dissected from the uterine walls, freed of decidua capsularis and Reichert's membrane, and the yolk sac was opened. For injection and electroporation, embryos were immobilised in a mould of agarose. Using a glass capillary controlled by a standard micromanipulator (Narishige MN-153, Japan) and connected to a pneumatic PicoPump (PV820, WPI), 0.3-0.6 µl (corresponding to about 1/10 of the lumenal volume of the anterior neural tube) of PBS containing 1-4 µg/µl plasmid DNA (pSVpaXΔ expressing β-gal (13), pEGFP-N2 expressing GFP (Clontech)) and esiRNAs (0.1-0.5 µg/µl) as indicated in the figure legends were injected into different regions of the neural tube or other cavities of the embryo. Immediately after injection, 5 square electrical pulses of 30 V, 50 ms each at 1s intervals, were delivered through platinum electrodes (7 mm diameter, 1 cm distance) mounted on plastic tweezers using a BTX ECM830 electroporator. The orientation of the electric field was used to direct the uptake of the nucleic acids to specific regions of the neural tube or other organs. After electroporation, embryos were placed in a whole-embryo culture incubator (Ikemoto, Japan) and allowed to continue their normal development for 24 h in 0.5 ml per embryo of a 2:1 mixture of immediately centrifuged, heat-inactivated mouse serum (Harlan) and DMEM in a continuous-flow atmosphere of 60%O₂, 5%CO₂, 35% N₂ (50 ml per min).

### Light microscopy

Embryos were fixed overnight at 4°C in 4% paraformaldehyde in 120 mM phosphate buffer pH 7.4, equilibrated in 30% sucrose in PBS and embedded in Tissue-Tek. Cryosections (10 µm) were prepared, permeabilised with 0.3% Triton X-100 in PBS, quenched with 10 mM NH₄Cl, and subjected to immunohistochemistry according to standard procedures using mouse monoclonal β-gal antibody (Sigma) and anti-mouse rhodamine-conjugated secondary antibody (Jackson ImmunoResearch) in PBS containing 5% BSA, 5%FCS and 0.1% Triton X-100. In some experiments, unfixed embryos were embedded in low-melting agarose, and vibratome sections (150 µm) were prepared, analyzed by fluorescence microscopy, fixed and processed for fluorescence microscopy of cryosections as above. Images were collected using version 3.5.1 IPlab software, and fluorescence of defined regions was quantified using NIHimage version 1.62.

### Results and Discussion

By monitoring GFP expression from a plasmid with a constitutive promoter (pEGFP-N2) as an indicator of cellular nucleic acid uptake, we first examined the organ- and region-specific delivery of nucleic acid upon injection into the lumen of the neural tube at defined positions, followed by electroporation with the electric field in a desired orientation (Fig. 1a). Indeed, analysis of E10 mouse embryos developing for a further 24 h in whole-embryo culture after the injection and electroporation showed that, depending on the segment of the neural tube injected and on the orientation of the electroporation electrodes, GFP expression could be directed to specific regions of the neuroepithelium (Fig. 1 b-e).

We then investigated the use of siRNA to achieve RNAi on transfected DNA. For this purpose, two expression vectors were used in combination, one (pEGFP-N2) driving GFP expression as a positive control and the other (pSVpaXΔ driving β-galactosidase (β-gal)expression as the target of RNAi. The two plasmids were coinjected into the lumen of the telencephalic neural tube of E10 mouse embryos, co-electroporated into the neuroepithelium, and the embryos allowed to continue normal development in whole-embryo culture for a further 24 h. Analysis of GFP fluorescence and β-gal immunoreactivity showed that the co-electroporation of the two reporter genes was highly efficient since almost all neuroepithelial cells expressing GFP also expressed β-gal (Fig. 2a-c, g).

Endoribonuclease-prepared siRNA (11) covering a 1365-nt stretch of the β-gal mRNA were generated.

When β-gal-directed esiRNA was included together with the GFP- and β-gal-expression plasmids in the injection and electroporation of the neural tube of E10 mouse embryos, β-gal expression as observed after 24 h of whole-embryo culture was abolished in almost all GFP-positive neuroepithelial cells (Fig. 2d-f, g). These results indicate the efficient delivery of esiRNA into neuroepithelial cells and the selective silencing of the targeted mRNA.

It was important to determine whether esiRNA and the present method of delivery to cells can be used to silence endogenous gene expression. For this purpose, we used (phenotypically wildtype) heterozygous embryos of a mouse knock-in line expressing GFP (carrying a nuclear localization signal) from the TIS21 locus. TIS21 is an antiproliferative gene that, during the development of the central nervous system, is specifically expressed in neuron-generating neuroepithelial cells (14), with an onset of expression in the mouse telencephalon at E10. Hence, to study mRNA silencing, TIS21 is superior to a gene constitutively expressed in the neural tube, whose translation product would be present already before RNAi and might persist, making an evaluation of the effects of siRNA difficult. We therefore investigated the potential use of esiRNA for silencing endogenous gene expression during mouse development by monitoring the appearance of GFP fluorescence in the neuroepithelium of E10 *tis21*^{+/GFP} mouse embryos.

We generated GFP-esiRNA covering virtually the entire coding sequence (712 nt) of GFP (excluding the nuclear localization signal) in the mRNA transcribed from the knocked-in TIS21 locus. This GFP-esiRNA was directed into neuroepithelial cells in specific regions of the developing E10 mouse brain by topical injection into the lumen of the anterior neural tube and directed electroporation as above. The orientation of the electrodes was such (cathode-right/anode-left) that the esiRNA would be electroporated selectively into neuroepithelial cells of the left half of the brain, with the right half serving as an internal positive control for TIS21 locus-driven GFP expression. Analysis of GFP fluorescence after 24 h of whole-embryo culture showed that, indeed, endogenous gene expression was prevented in those segments of the neuroepithelium targeted by the site of esi-RNA injection (telencephalon or diencephalon), and that the silencing was selective for the left, anode-facing half of the brain (Fig. 3a-d). As judged from the GFP fluorescence per unit area, silencing in the most affected regions was almost complete (Fig. 3e) and even for the entire telencephalon or diencephalon was at least 70% efficient (data not shown). Given that the fluorescence per unit area is the product of the amount of fluorescence per cell times the number of fluorescent cells per unit area, the latter was also quantitated. This revealed a dramatic decrease in the number of fluorescent neuroepithelial cells per unit area in the affected reaion compared to the corresponding region on the contralateral side (Fig. 3f-h). However, we also observed that the esiRNA-triggered decrease in the number of fluorescent neuroepithelial cells (Fig. 3h) was slightly less than the reduction in the amount of fluorescence per unit area (Fig. 3e). Consistent with this, the few neuroepithelial cells in a silenced region that still expressed GFP appeared to show, on average, a lower level of fluorescence than the control GFP-expressing neuroepithelial cells, i.e. those in the corresponding region on the contralateral side.

The percentage of silenced neuroepithelial cells observed upon esiRNA-triggered RNAi of endogenous gene expression (Fig. 3h) appeared to be greater than expected from the proportion of neuroepithelial cells transfected upon electroporation of the reporter plasmids, which was not more than half of the neuroepithelial cells in a given area (Fig. 2a,b). This may reflect a greater electroporation efficiency because the small esiRNAs may enter cells more easily than large plasmid DNA. Altematively, the high proportion of silenced cells raises the possibility that some kind of cell-to-cell propagation mechanism of RNAi may exist in mammals, as is known to be the case for systemic RNAi in C. elegans (1, 15).

Our results establish the use of esiRNA for specific mRNA silencing in the mouse embryo, and document an approach to perform siRNA-triggered RNAi in a tissue-specific manner. They also and in a surprising manner extend recent reports (16, 17) showing siRNA-triggered RNAi in adult mice that appeared while the present work was being prepared for publication. Long dsRNA, which trigger the interferon response in most mammalian cells (4), have successfully been used for RNAi only in mouse oocytes and preimplantation embryos (18-20) due to the absence of the interferon response at these early developmental stages. The present demonstration of tissue-specific siRNA-triggered RNAi in postimplantation mouse embryos suggests that this powerful method can be used for investigating the function of individual genes in mammalian development. Our data showing that the knocking-down of the protein of interest in the targeted cell population (the neuroepithelial cells) is dramatic (≈90% on average, with the majority of cells showing no detectable expression), but not complete, as would be the case in a knock-out mouse, does not invalidate this conclusion because there are numerous examples in which this degree of reduction in gene product has led to a loss-of-function phenotype (21).

Several aspects of the present experimental approach deserve comment. First, it should be noted that our combination of topical injection of esiRNA, directed electroporation and whole-embryo culture as an approach to trigger, and observe the effects of, tissue-specific RNAi is not restricted to the developing central nervous system but can, in principle, be applied to various organs of the mouse embryo including those lacking intemal cavities. In a separate set of experiments, we injected pEGFP-N2 into the cavity of the developing heart of E10 mouse embryos, without or with GFP-directed esi-RNA, followed by electroporation and whole-embryo culture for 24 h. This procedure did not have any obvious effects on heart development and functionality. The presence of GFP-directed esiRNA prevented the GFP expression observed in the control heart (data not shown). In addition, nucleic acids have successfully been delivered into target cells by injection directly into the tissue (e.g., muscle) rather than into a cavity surrounded by tissue, followed by electroporation (22).

Second, the present experimental approach is applicable at various developmental stages, since the start of whole-embryo culture of mouse embryos can be any time point between E7 and E12 (12). Moreover, the current limitation of the whole-embryo culture period to about two days does not preclude the use of electroporation for tissue-specific delivery of siRNA in long-term studies of mouse development, because electroporation of mouse embryos can also be performed in utero (23, 24). In this context, although the duration of esiRNA-triggered RNAi in the mouse embryo remains to be determined, this process lasted for up to 10 days in mammalian cell lines (11), and in adult mice efficient silencing was seen for at least 4 days post siRNA administration (17). In addition the present invention comprises the use of shRNA instead of siRNA in all above applications including the combination of topical injection, directed electroporation and whole-embryo culture for the delivery of DNA templates expressing short hairpin RNAs (shRNA) (10, 25, 26).

Third, the present invention contemplates that mixtures of esiRNAs, targeting the mRNAs of various proteins, can be used at the same time. With the present approach, genome-wide functional screens, such as those initiated in C. elegans using long dsRNA (2, 3), will now be possible in the mouse to systematically investigate not only neurogenesis but also other aspects of mammalian development.

### References

1. Fire, A., Xu, S., Montgomery, M. K., Kostas, S. A., Driver, S. E. & Mello, C. C. (1998) *Nature* **391,** 806-811.
2. Fraser, A. G., Kamath, R. S., Zipperlen, P., Martinez-Campos, M., Sohrmann, M. & Ahringer, J. (2000) *Nature* **408,** 325-330.
3. Gönczy, P., Echeverri, C., Oegema, K., Coulson, A., Jones, S. J., Copley, R. R., Duperon, J., Oegema, J., Brehm, M., Cassin, E., Hannak, E., Kirkham, M., Pichler, S., Flohrs, K., Goessen, A., Leidel, S., Alleaume, A. M., Martin; C., Ozlu, N., Bork, P. & Hyman, A. A. (2000) *Nature* **408,** 331-336.
4. Hannon, G. J. (2002) *Nature* **418,** 244-51.
5. Schmid, A., Schindelholz, B. & Zinn, K. (2002) *Trends Neurosci* **25,** 71-4.
6. Wang, M. B. & Waterhouse, P. M. (2002) *Curr Opin Plant Biol* **5,** 146-50.
7. Stark, G. R., Kerr, I. M., Williams, B. R., Silverman, R. H. & Schreiber, R. D. (1998) *Annu. Rev. Biochem.* **67,** 227-264.
8. Elbashir, S. M., Harborth, J., Lendeckel, W., Yalcin, A., Weber, K. & Tuschl, T. (2001) *Nature* **411,** 494-498.
9. Elbashir, S. M., Harborth, J., Weber, K. & Tuschl, T. (2002) *Methods* **26,** 199-213.
10. Brummelkamp, T. R., Bernards, R. & Agami, R. (2002) *Science* **296,** 550-553.
11. Yang, D., Buchholz, F., Huang, Z., Goga, A., Chen, C. Y., Brodsky, F. M. & Bishop, J. M. (2002) *Proc. Natl. Acad. Sci. USA* **99,** 9942-9947.
12. Cockroft, D. L. (1990) in *Postimplantation mammalian embryos,* eds. Copp, A. J. & Cockroft, D. L. (IRL - Press, Oxford, New York, Tokyo), pp. 15-40.
13. Buchholz, F., Ringrose, L., Angrand, P. O., Rossi, F. & Stewart, A. F. (1996) *Nucleic Acids Res.* **24,** 4256-4562.
14. lacopetti, P., Michelini, M., Stuckmann, I., Oback, B., Aaku-Saraste, E. & Huttner, W. B. (1999) *Proc. Natl. Acad. Sci. USA* **96,** 4639-4644.
15. Winston, W. M., Molodowitch, C. & Hunter, C. P. (2002) *Science* **295,** 2456-2459.
16. McCaffrey, A. P., Meuse, L., Pham, T. T., Conklin, D. S., Hannon, G. J. & Kay, M. A. (2002) *Nature* **418,** 38-39.
17. Lewis, D. L., Hagstrom, J. E., Loomis, A. G., Wolff, J. A. & Herweijer, H. (2002) *Nature Genet.* **32,** in press.
18. Wianny, F. & Zernicka-Goetz, M. (2000) *Nature Cell Biol.* **2,** 70-75.
19. Zernicka-Goetz, M. (2000) *Nature* **405,** 733.
20. Svoboda, P., Stein, P., Hayashi, H. & Schultz, R. M. (2000) *Development* **127**, 4147-4156.
21. Bargmann, C. I. (2001) *Genome Biol* **2.**
22. Aihara, H. & Miyazaki, J. (1998) *Nat Biotechnol* **16,** 867-70.
23. Tabata, H. & Nakajima, K. (2001) *Neuroscience* **103,** 865-872.
24. Saito, T. & Nakatsuji, N. (2001) *Dev. Biol.* **240,** 237-246.
25. Paddison, P. J., Caudy, A. A., Bernstein, E., Hannon, G. J. & Conklin, D. S. (2002) *Genes Dev* **16,** 948-958.
26. Tuschl, T. (2002) *Nat Biotechnol* **20,** 446-8.

## Claims

1. A method of interfering with gene expression in a mammalian post-implantation embryo comprising the step of introducing siRNA molecules or shRNA molecules or DNA molecules encoding siRNA or shRNA molecules wherein said siRNA molecules or said shRNA molecules have a region of identity or of substantial identity of at least 15 nucleotides with the gene(s) the expression of which is to be interfered with into cells of said post-implantation embryo.

2. The method of claim 1 wherein said mammalian post-implantation embryo is a human embryo, a non-human primate embryo, a dog, cat, pig, cow, horse, donkey, goat, sheep, rabbit, rat or a mouse embryo.

3. The method of claim 1 or 2 wherein said post-implantation embryo is in the stage between implantation in the wall of the uterus and birth.

4. The method of any one of claims 1 to 3 wherein the introduction is effected by injection into the embryo.

5. The method of claim 4, wherein injection is followed by electroporation or nucleic acid transfection.

6. The method of any one of claims 1 to 5 wherein the siRNA has been prepared by endoribonuclease-digestion.

7. The method of any one of claims 1 to 6 wherein the siRNAs have a maximum length of about 40 bp.

8. The method of any one of claims 1 to 7 wherein a variety of siRNA molecules or shRNA molecules or DNA molecules encoding a variety of siRNA or shRNA molecules are introduced wherein different siRNA molecules have a region of identity or of substantial identity of at least 15 bp with different genes.

9. The method of any one of claims 1 to 8 wherein the gene(s) the expression of which is interfered with is/are selected from the group of developmental genes or genes involved in the generation of cancer.

10. The method of any one of claims 1 to 9 wherein said mammalian post-implantation embryo is cultured on a mammalian whole-embryo culture or in the uterus.

11. The method of any one of claims 1 to 10 wherein said siRNA or said shRNA is chemically modified or derivatised.

12. A method of performing a genomic-wide functional screen comprising introducing a variety of siRNA molecules or shRNA molecules or DNA molecules encoding a variety of siRNA or shRNA molecules wherein different siRNA or shRNA molecules have a region of identity or substantial identity of at least 15 bp with different genes into a mammalian non-human post-implantation embryo.


### Annex to the application documents-subsequently filed sequences listing

SEQUENCE LISTING

<110>  Max-Planck-Gesellschaft zur Förderung der Wissenschaften e.V.

<120>  Method of interfering with gene expression in mammalian post-implantation
embryos

<130>  G2447EP

<140>  EP 03 02 2984.3

<141>  2003-10-10

<150>  EP 02 02 2922.5

<151>  2002-10-10

<160>  4

<170>  PatentIn version 3.1

<210>  1

<211>  44

<212>  DNA

<213>  artificial sequence

<220>

<223>  /note="Description of artificial sequence: primer"

<400>  1
taatacgact cactataggg agaatcgtaa tcacccgagt gtga                    44

<210>  2

<211>  41

<212>  DNA

<213>  artificial sequence

<220>

<223>  /note="Description of artificial sequence: primer"

<400>  2
aattaccctc actaaaggga gccctaatcc gagccagttt a                       41

<210>  3

<211>  36

<212>  DNA

<213>  artificial sequence


<220>

<223>  /note="Description of artificial sequence: primer"

<400>  3
ttaatacgac tcactatagg tgagcaaggg cgagga                                36


<210>  4

<211>  40

<212>  DNA

<213>  artificial sequence


<220>

<223>  /note="Description of artificial sequence: primer"

<400>  4
taattaaccc tcactaaagg gtacagctcg tccatgccga                            40